(19) Europäisches Patentamt
European Patent Office
Office européen des brevets

(11) Veröffentlichungsnummer: **0 356 828 A2**

(12) **EUROPÄISCHE PATENTANMELDUNG**

(21) Anmeldenummer: **89115216.7**

(22) Anmeldetag: **18.08.89**

(51) Int. Cl.⁵: **C07D 251/46 , A01N 47/36 , //C07D251/16**

(30) Priorität: **31.08.88 DE 3829469**

(43) Veröffentlichungstag der Anmeldung: **07.03.90 Patentblatt 90/10**

(84) Benannte Vertragsstaaten: **BE CH DE FR GB IT LI NL**

(71) Anmelder: **BAYER AG**
**D-5090 Leverkusen 1 Bayerwerk(DE)**

(72) Erfinder: **Riebel, Hans-Jochem, Dr.**
**In der Beek 92**
**D-5600 Wuppertal 1(DE)**

(54) **Verfahren zur Herstellung von Sulfonylisothioharnstoffen.**

(57) Herbizid wirksame Sulfonylisothioharnstoffe der allgemeinen Formel (I)

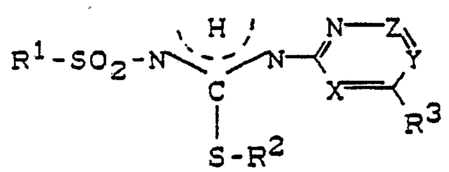

(I)

in welcher
$R^1$ für einen gegebenenfalls substituierten Rest aus der Reihe Alkyl, Aralkyl, Aryl und Heteroaryl steht,
$R^2$ für jeweils gegebenenfalls substituiertes Alkyl oder Aralkyl steht,
$R^3$ für Wasserstoff, Halogen, Hydroxy, Amino oder einen gegebenenfalls substituierten Rest aus der Reihe Alkyl, Alkoxy, Alkylthio, Alkylamino und Dialkylamino steht,
X für Stickstoff oder eine CH-Gruppierung steht,
Y für Stickstoff oder eine $CR^4$-Gruppierung steht,
und
Z für Stickstoff oder eine $CR^5$-Gruppierung steht,
(wobei $R^4$ und $R^5$ die in der Beschreibung angegebenen Bedeutungen haben),
erhält man auf einfache Weise in hohen Ausbeuten, indem man Sulfonsäureamide der allgemeinen Formel (II)

$R^1 - SO_2 - NH_2$ (II)

- oder Metallsalze von Verbindungen der Formel (II) -
mit N-Heteroaryl-iminodithiokohlensäure-S,S-diestern der allgemeinen Formel (III)

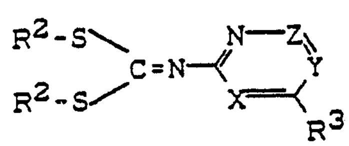

(III)

gegebenenfalls in Gegenwart eines Säureakzeptors und in Gegenwart eines Verdünnungsmittels bei Temperaturen zwischen 20°C und 200°C umsetzt.

EP 0 356 828 A2

Die neuen Zwischenprodukte der Formel (III) und ein Verfahren zu ihrer Herstellung sind ebenfalls Gegenstand der Erfindung.

## Verfahren zur Herstellung von Sulfonylisothioharnstoffen

Die Erfindung betrifft ein neues Verfahren zur Herstellung von bekannten Sulfonylisothioharnstoffen und neue Zwischenprodukte hierfür.

Es ist bekannt, daß man Sulfonylisothioharnstoffe erhält, wenn man Sulfonylthioharnstoffe in Salze überführt und diese mit Alkylierungsmitteln umsetzt (vgl. EP-A 5 986).

Für die Herstellung der hierbei als Ausgangsstoffe benötigten Sulfonylthioharnstoffe werden mehrere Reaktionsschritte benötigt: Zunächst werden entsprechende Sulfonsäureamide mit Schwefelkohlenstoff (Carbondisulfid) und Kaliumhydroxid zu Dikaliumsalzen von Sulfonyliminodithiokohlensäuren umgesetzt; deren Umsetzung mit Phosgen führt zu Sulfonylisothiocyanaten und aus letzteren er hält man mit Aminoverbindungen entsprechende Sulfonylthioharnstoffe. Dieser bekannte Syntheseweg zu Sulfonylisothioharnstoffen erfordert in einzelnen Stufen hohen apparativen Aufwand und führt zudem hinsichtlich Ausbeute und Qualität der Produkte nicht immer zu befriedigenden Ergebnissen.

Es wurde nun gefunden, daß man Sulfonylisothioharnstoffe der allgemeinen Formel (I)

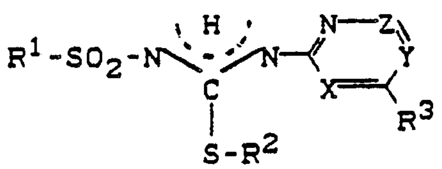

(I)

in welcher

$R^1$ für einen gegebenenfalls substituierten Rest aus der Reihe Alkyl, Aralkyl, Aryl und Heteroaryl steht,

$R^2$ für jeweils gegebenenfalls substituiertes Alkyl oder Aralkyl steht,

$R^3$ für Wasserstoff, Halogen, Hydroxy, Amino oder einen gegebenenfalls substituierten Rest aus der Reihe Alkyl, Alkoxy, Alkylthio, Alkylamino und Dialkylamino steht,

X für Stickstoff oder eine CH-Gruppierung steht,

Y für Stickstoff oder eine $CR^4$-Gruppierung steht, worin

$R^4$ für Wasserstoff, Halogen, Cyano, Alkyl, Formyl, Alkylcarbonyl oder Alkoxycarbonyl steht, und

Z für Stickstoff oder eine $CR^5$-Gruppierung steht, worin

$R^5$ für Wasserstoff, Halogen, Hydroxy, Amino oder einen gegebenenfalls substituierten Rest aus der Reihe Alkyl, Alkoxy, Alkylthio, Alkylamino und Dialkylamino steht,

auf einfache Weise in hohen Ausbeuten erhält,

wenn man Sulfonsäureamide der allgemeinen Formel (II)

$R^1 - SO_2 - NH_2$ (II)

in welcher

$R^1$ die oben angegebene Bedeutung hat,

- oder Metallsalze von Verbindungen der Formel (II) -

mit N-Heteroaryl-iminodithiokohlensäure-S,S-diestern der allgemeinen Formel (III)

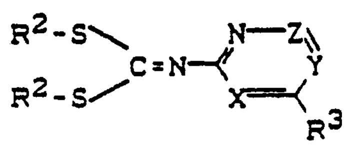

(III)

in welcher

$R^2$, $R^3$, X, Y und Z die oben angegebenen Bedeutungen haben,

gegebenenfalls in Gegenwart eines Säureakzeptors und in Gegenwart eines Verdünnungsmittels bei Temperaturen zwischen 20 °C und 200 °C umsetzt.

Überraschenderweise gelingt es mit Hilfe des erfindungsgemäßen Verfahrens, die Verbindungen der Formel (I) auf relativ einfache Weise in hohen Ausbeuten herzustellen.

Verwendet man beispielsweise 2-Fluor-benzolsulfonsäureamid und N-(4,6-Dimethoxy-pyrimidin-2-yl)-iminodithiokohlensäure-S,S-dimethylester als Ausgangsstoffe, so kann der Reaktionsablauf durch das folgende Formelschema skizziert werden:

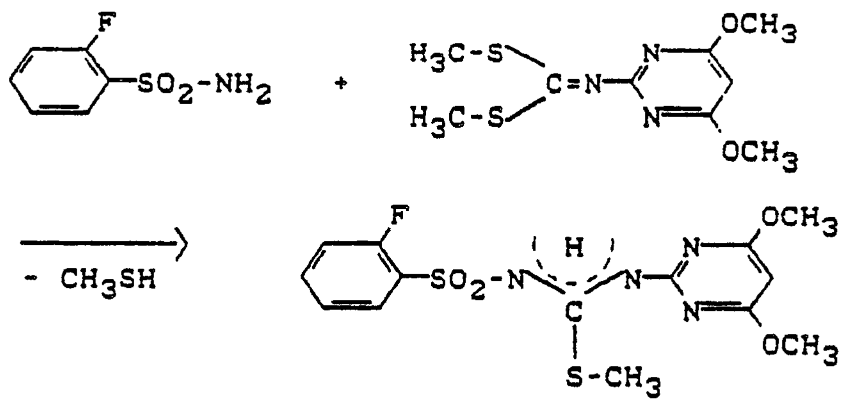


Die als Ausgangsstoffe zu verwendenden Sulfonsäureamide sind durch die Formel (II) allgemein definiert. In Formel (II) steht vorzugsweise $R^1$ für den Rest

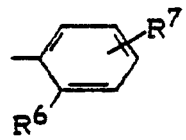
,

worin
$R^6$ für Wasserstoff, Halogen, Cyano, Nitro, $C_1$-$C_4$-Alkyl (welches gegebenenfalls durch Halogen, Cyano, $C_1$-$C_4$-Alkoxy oder $C_1$-$C_4$-Alkoxy-carbonyl substituiert ist), $C_1$-$C_4$-Alkoxy (welches gegebenenfalls durch Halogen, Cyano, $C_1$-$C_4$-Alkoxy oder $C_1$-$C_4$-Alkoxy-carbonyl substituiert ist), $C_1$-$C_4$-Alkylthio, $C_1$-$C_4$-Alkylsulfinyl oder $C_1$-$C_4$-Alkylsulfonyl (welche gegebenenfalls durch Halogen, Cyano, $C_1$-$C_4$-Alkoxy oder $C_1$-$C_4$-Alkoxy-carbonyl substituiert sind), für Di-($C_1$-$C_4$-alkyl)-aminosulfonyl, N-($C_1$-$C_4$-Alkoxy)-N-$C_1$-$C_4$-alkyl-aminosulfonyl, Phenyl, Phenoxy, $C_1$-$C_4$-Alkoxy-carbonyl oder Di-($C_1$-$C_4$-alkylamino)-carbonyl steht und
$R^7$ für Wasserstoff oder Halogen steht,
oder es steht vorzugsweise
$R^1$ für den Rest

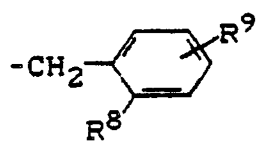
,

worin
$R^8$ für Wasserstoff, Halogen, Cyano, Nitro, $C_1$-$C_4$- Alkyl (welches gegebenenfalls durch Halogen, Cyano, $C_1$-$C_4$-Alkoxy oder $C_1$-$C_4$-Alkoxy-carbonyl substituiert ist), $C_1$-$C_4$-Alkoxy (welches gegebenenfalls durch Halogen, Cyano, $C_1$-$C_4$-Alkoxy oder $C_1$-$C_4$-Alkoxy-carbonyl substituiert ist), $C_1$-$C_4$-Alkylthio, $C_1$-$C_4$-Alkylsulfinyl oder $C_1$-$C_4$-Alkylsulfonyl (welche gegebenenfalls durch Halogen, Cyano, $C_1$-$C_4$-Alkoxy oder $C_1$-$C_4$-Alkoxy-carbonyl substituiert sind), für Di-($C_1$-$C_4$-alkyl)-aminosulfonyl oder $C_1$-$C_4$-Alkoxy-carbonyl steht und
$R^9$ für Wasserstoff oder Halogen steht,
oder es steht vorzugsweise
$R^1$ für den Rest

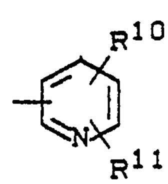
,

worin
$R^{10}$ für Wasserstoff, Halogen, Cyano, Nitro, $C_1$-$C_4$-Alkyl (welches gegebenenfalls durch Halogen substituiert

ist), $C_2$-$C_4$-Alkenyl (welches gegebenenfalls durch Halogen substituiert ist), $C_1$-$C_4$-Alkoxy (welches gegebenenfalls durch Halogen substituiert ist), $C_1$-$C_4$-Alkylthio, $C_1$-$C_4$-Alkylsulfinyl oder $C_1$-$C_4$-Alkylsulfonyl (welche gegebenenfalls durch Halogen substituiert sind), Di-($C_1$-$C_4$-alkyl)-aminosulfonyl, $C_1$-$C_4$-Alkoxycarbonyl, Di-($C_1$-$C_4$-alkyl)-aminocarbonyl oder Dioxolanyl steht und
$R^{11}$ für Wasserstoff oder Halogen steht,
oder es steht vorzugsweise
$R^1$ für den Rest

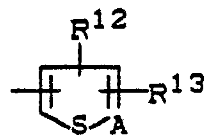
,

worin
A für Stickstoff oder eine CH-Gruppierung steht,
$R^{12}$ für Wasserstoff, Halogen, Cyano, Nitro, $C_1$-$C_4$-Alkyl (welches gegebenenfalls durch Halogen, $C_1$-$C_4$-Alkoxy oder $C_1$-$C_4$-Halogenalkoxy substituiert ist), $C_1$-$C_4$-Alkoxy (welches gegebenenfalls durch Halogen oder $C_1$-$C_4$-Alkoxy substituiert ist), $C_1$-$C_4$-Alkylthio, $C_1$-$C_4$-Alkylsulfinyl oder $C_1$-$C_4$-Alkylsulfonyl (welche gegebenenfalls durch Halogen substituiert sind), Di-($C_1$-$C_4$-alkyl)-aminosulfonyl oder $C_1$-$C_4$-Alkoxycarbonyl steht und
$R^{13}$ für Wasserstoff oder Halogen steht,
oder es steht vorzusweise
$R^1$ für den Rest

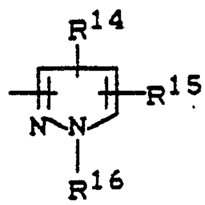
,

worin
$R^{14}$ für Wasserstoff, Halogen, Cyano, Nitro, $C_1$-$C_4$-Alkyl (welches gegebenenfalls durch Halogen substituiert ist), $C_1$-$C_4$-Alkoxy (welches gegebenenfalls durch Halogen substituiert ist), Dioxolanyl oder $C_1$-$C_4$-Alkoxycarbonyl steht,
$R^{15}$ für Wasserstoff oder Halogen steht und
$R^{16}$ für Wasserstoff, $C_1$-$C_4$-Alkyl, Phenyl oder (Iso)Chinolinyl steht,
oder es steht vorzugsweise
$R^1$ für den Rest

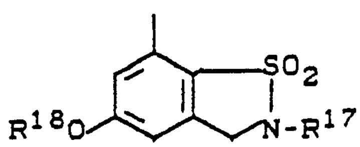
,

worin
$R^{17}$ und $R^{18}$ für $C_1$-$C_4$-Alkyl stehen,
oder es steht vorzugsweise
$R^1$ für den Rest

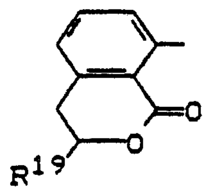,

worin
$R^{19}$ für Wasserstoff oder Methyl steht.

Als Ausgangsstoffe besonders bevorzugt werden die Verbindungen der Formel (II), in welcher $R^1$ für den Rest

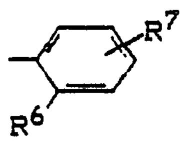,

worin
$R^6$ für Fluor, Chlor, Brom, Methyl, Trifluormethyl, Methoxy, Difluormethoxy, Trifluormethoxy, Methylthio, Methylsulfonyl, Dimethylaminosulfonyl, N-Methoxy-N-methyl-aminosulfonyl, Phenyl, Methoxycarbonyl oder Ethoxycarbonyl steht und
$R^7$ für Wasserstoff, Fluor oder Chlor steht.

Als Beispiele für die Ausgangsstoffe der Formel (II) seien genannt:
2-Fluor-, 2-Chlor-, 2-Brom-, 2,5-Dichlor-, 2,6-Dichlor-, 2-Methyl-, 2-Trifluormethyl-, 2-Methoxy-, 2-Difluormethoxy-, 2-Trifluormethoxy-, 2-Methylthio-, 2-Methylsulfonyl-, 2-Dimethylaminosulfonyl-, 2-(N-Methoxy-N-methylamino)-sulfonyl-, 2-Phenyl-, 2-Methoxycarbonyl- und 2-Ethoxycarbonyl-benzolsulfonsäureamid.

Die Ausgangsstoffe der Formel (II) sind bekannt und/oder können nach an sich bekannten Verfahren hergestellt werden (vgl. US-P 4 371 391; J. Org. Chem. 27 (1962), 1703 - 1709; US-P 4 310 346; US-P 4 452 628; EP-A 44 808; EP-A 87 780; US-P 4 732 711; EP-A 271 780).

Die weiter als Ausgangsstoffe einzusetzenden N-Heteroaryl-iminodithiokohlensäure-S,S-diester sind durch die Formel (III) allgemein definiert. In Formel (III) steht vorzugsweise
$R^2$ für $C_1$-$C_6$-Alkyl (welches gegebenenfalls durch Fluor, Chlor, Cyano, $C_1$-$C_2$-Alkoxy, Carboxy, $C_1$-$C_2$-Alkoxy-carbonyl, Aminocarbonyl, $C_1$-$C_2$-Alkylaminocarbonyl oder Di-($C_1$-$C_2$-alkyl)-amino-carbonyl substituiert ist) oder für Benzyl oder Phenylethyl (welche im Phenylteil gegebenenfalls durch Fluor, Chlor, $C_1$-$C_2$-Alkyl, $C_1$-$C_2$-Halogenalkyl, $C_1$-$C_2$-Alkoxy, $C_1$-$C_2$-Halogenalkoxy oder $C_1$-$C_2$-Alkoxy-carbonyl substituiert sind),
$R^3$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino, Dimethylamino oder Diethylamino,
X für Stickstoff oder eine CH-Gruppierung,
Y für Stickstoff oder eine $CR^4$-Gruppierung, worin
$R^4$ für Wasserstoff, Fluor, Chlor, Brom, Cyano, Methyl, Formyl, Acetyl, Methoxycarbonyl oder Ethoxycarbonyl steht und
Z für Stickstoff oder eine $CR^5$-Gruppierung, worin
$R^5$ für Wasserstoff, Fluor, Chlor, Brom, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkylamino, Dimethylamino oder Diethylamino steht.

Als Ausgangsstoffe besonders bevorzugt werden die Verbindungen der Formel (III), in welcher
$R^2$ für Methyl, Ethyl, Carboxymethyl, Methoxycarbonylmethyl, Ethoxycarbonylmethyl oder Benzyl steht,
$R^3$ für Wasserstoff, Fluor, Chlor, Brom, Methyl, Ethyl, Trifluormethyl, Methoxy, Ethoxy, Difluormethoxy, Methylthio, Ethylthio, Amino, Methylamino, Ethylamino, Dimethylamino oder Diethylamino steht,
X für Stickstoff oder eine CH-Gruppierung steht,
Y für Stickstoff oder eine $CR^4$-Gruppierung steht, worin
$R^4$ für Wasserstoff, Fluor, Chlor oder Methyl steht, und
Z für Stickstoff oder eine $CR^5$-Gruppierung steht, worin
$R^5$ für Wasserstoff, Fluor, Chlor, Brom, Methyl, Ethyl, Trifluormethyl, Methoxy, Ethoxy, Propoxy, Isopropoxy,

Difluormethoxy, Methylthio, Ethylthio, Methylamino, Ethylamino, Dimethylamino oder Diethylamino steht.

Beispiele für die Ausgangsstoffe der Formel (III) sind in der nachstehenden Tabelle 1 aufgeführt.

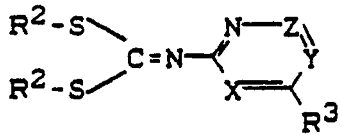

(III)

Tabelle 1: Beispiele für die Ausgangsstoffe der Formel (III)

| $R^2$ | $R^3$ | X | Y | Z |
|---|---|---|---|---|
| $CH_3$ | $CH_3$ | N | CH | C-$CH_3$ |
| $CH_3$ | $CH_3$ | N | CH | C-$OCH_3$ |
| $CH_3$ | $OCH_3$ | N | CH | C-$OCH_3$ |
| $CH_3$ | $OCH_3$ | N | CH | C-Cl |
| $CH_3$ | H | N | CH | C-$CH_3$ |
| $CH_3$ | $CF_3$ | N | CH | C-$OCH_3$ |
| $CH_3$ | $OCH_3$ | N | CH | C-$OCHF_2$ |
| $CH_3$ | $CH_3$ | N | CH | C-$OCHF_2$ |
| $CH_3$ | $CH_3$ | N | N | C-$CH_3$ |
| $CH_3$ | $CH_3$ | N | N | C-$OCH_3$ |
| $CH_3$ | $OCH_3$ | N | N | C-$OCH_3$ |
| $CH_3$ | $C_2H_5$ | N | CH | C-$OCH_3$ |
| $CH_3$ | $CH_3$ | N | N | C-$OC_2H_5$ |
| $CH_3$ | $C_2H_5$ | N | N | C-$OCH_3$ |
| $CH_3$ | $CH_3$ | N | N | C-Cl |
| $CH_3$ | $CH_3$ | CH | N | C-$CH_3$ |
| $CH_3$ | $OCHF_2$ | N | CH | C-$OCHF_2$ |
| $CH_3$ | $OCH_3$ | CH | N | C-$OCH_3$ |

Tabelle 1 - Fortsetzung

| $R^2$ | $R^3$ | X | Y | Z |
|---|---|---|---|---|
| $CH_3$ | $CH_3$ | N | CH | $C\text{-}SCH_3$ |
| $CH_3$ | $CH_3$ | N | CH | $C\text{-}N(CH_3)_2$ |
| $CH_3$ | $OCH_3$ | N | CH | $C\text{-}SCH_3$ |
| $CH_3$ | $OCH_3$ | N | N | $C\text{-}NHC_2H_5$ |
| $CH_3$ | $OC_2H_5$ | N | N | $C\text{-}NHCH_3$ |
| $CH_3$ | $CH_3$ | CH | CH | $C\text{-}CH_3$ |

Die Ausgangsstoffe der Formel (III) sind noch nicht aus der Literatur bekannt.

Man erhält die neuen N-Heteroaryl-iminodithiokohlensäure-S,S-diester der allgemeinen Formel (III), wenn man Aminohetarene der allgemeinen Formel (IV)

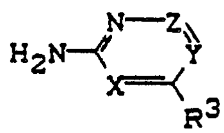

(IV)

in welcher

$R^3$, X, Y und Z die oben angegebenen Bedeutungen haben,

- oder Metallsalze von Verbindungen der Formel (IV) -

mit Schwefelkohlenstoff (Carbondisulfid) in Gegenwart einer starken Base, wie z. B. Natrium- oder Kaliumhydroxid, und in Gegenwart eines Verdünnungsmittels, wie z. B. Dimethylformamid und/oder Wasser, bei Temperaturen zwischen -20 °C und +50 °C umsetzt und in situ weiter mit Alkylierungsmitteln der allgemeinen Formel (V)

$R^2 - X^1$ (V)

in welcher

$R^2$ die oben angegebene Bedeutung hat und

$X^1$ für Chlor, Brom oder Iod steht,

bei Temperaturen zwischen 0 °C und 100 °C umsetzt. Die nach Verdünnen mit Wasser kristallin anfallenden Produkte der Formel (III) können durch Absaugen isoliert werden.

Die als Zwischenprodukte zu verwendenden Aminohetarene sind durch die Formel (IV) allgemein definiert. In Formel (IV) haben $R^3$, X, Y und Z vorzugsweise bzw. insbesondere diejenigen Bedeutungen, die bereits oben im Zusammenhang mit der Beschreibung der erfindungsgemäßen Verbindungen der Formel (III) vorzugsweise bzw. als besonders bevorzugt für $R^3$, X, Y und Z angegeben wurden.

Als Beispiele für die Verbindungen der Formel (IV) seien genannt:

2-Amino-4,6-dimethyl-pyrimidin, -4-methyl-6-methoxy-pyrimidin, -4,6-dimethoxy-pyrimidin, -4-methyl-6-ethoxy-pyrimidin, -4-chlor-6-methoxy-pyrimidin, -4-methyl-pyrimidin, -4-chlor-6-methyl-pyrimidin, -4-trifluormethyl-6-methoxy-pyrimidin, -4-methoxy-6-difluormethoxy-pyrimidin, -4-methyl-6-difluormethoxy-pyrimidin, -4,6-bis-difluor-methoxy-pyrimidin, -4-chlor-6-ethoxy-pyrimidin, -4,6-diethoxy-pyrimidin, -4,5-dichlor-6-methyl-pyrimidin, -4-methyl-5-chlor-6-methoxy-pyrimidin, -4,6-dichlor-pyrimidin, -4-ethyl-6-methoxy-pyrimidin, -5-chlor-4,6-dimethoxy-pyrimidin sowie -4,6-bis-trifluormethyl-pyrimidin, ferner 2-Amino-4,6-dimethyl-s-triazin, -4-methyl-6-methoxy-s-triazin, -4,6-dimethoxy-s-triazin, -4-ethyl-6-methoxy-s-triazin und -4-methyl-6-ethoxy-s-triazin.

Die Aminohetarene der Formel (IV) sind bekannt und/oder können nach an sich bekannten Verfahren hergestellt werden (vgl. Chem. Pharm. Bull. 11 (1963), 1382; US-P 4 299 960; EP-A 121 082; EP-A 125 205; EP-A 126 711; EP-A 152 378; EP-A 158 594).

Die weiter als Zwischenprodukte benötigten Alkylierungsmittel sind durch die Formel (V) allgemein

definiert. In Formel (V) hat $R^2$ vorzugsweise bzw. insbesondere diejenige Bedeutung, die bereits oben im Zusammenhang mit der Beschreibung der erfindungsgemäßen Verbindungen der Formel (III) vorzugsweise bzw. als besonders bevorzugt für $R^2$ angegeben wurde und $X^1$ steht auch vorzugsweise für Chlor, Brom oder Iod.

Als Beispiele für die Alkylierungsmittel der Formel (V) seien genannt:
Methyl- und Ethyl-chlorid, -bromid und -iodid, Chlor-und Brom-essigsäure sowie deren Methylester und Ethylester, Benzyl-chlorid und -bromid.

Die Verbindungen der Formel (V) sind bekannte organische Synthesechemikalien.

Das erfindungsgemäße Verfahren zur Herstellung der Sulfonylisothioharnstoffe der Formel (I) wird vorzugsweise unter Verwendung von Verdünnungsmitteln durchgeführt. Als Verdünnungsmittel kommen dabei praktisch alle üblichen organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole, wie Methanol, Ethanol, Propanol, Isopropanol, Butanol, Isobutanol, sec-Butanol, tert-Butanol, Pentanol, Isopentanol, sec-Pentanol und tert-Pentanol, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kalium-methylat, -ethylat, -propylat, -isopropylat, -butylat, -isobutylat, -sec-butylat und -tert-butylat sowie Natrium-, Kalium- und Calcium-hydrid.

Das erfindungsgemäße Verfahren wird im allgemeinen bei Temperaturen zwischen 20 °C und 200 °C, vorzugsweise zwischen 60 °C und 140 °C, und im allgemeinen bei Normaldruck durchgeführt.

Zur Durchführung des erfindungsgemäßen Verfahrens können die Ausgangsstoffe in verschiedenen Molverhältnissen eingesetzt werden. Ein Überschuß der einen oder anderen Komponente bringt jedoch keine wesentlichen Vorteile. Die Ausgangsstoffe werden daher vorzugsweise in etwa äquimolaren Mengen eingesetzt.

Die Ausgangsstoffe können in beliebiger Reihenfolge zusammengegeben werden. In einer bevorzugten Ausführungsform des erfindungsgemäßen Verfahrens wird zunächst aus einem Sulfonsäureamid der Formel (II) und einem der oben angegebenen Säureakzeptoren, vorzugsweise in Gegenwart eines der oben angegebenen Verdünnungsmittel, ein Metallsalz der Verbindung der Formel (II) erzeugt. Hierzu wird dann ein N-Heteroaryl-iminodithiokohlensäure-S,S-diester der Formel (III) gegeben und das Reaktionsgemisch wird bei der jeweils erforderlichen Temperatur bis zum Ende der Umsetzung gerührt.

Die Aufarbeitung kann nach üblichen Methoden durchgeführt werden. Im allgemeinen wird nach Abkühlen auf Temperaturen zwischen 0 °C und 20 °C mit einer wäßrigen Protonensäure, wie z B. Salzsäure oder Schwefelsäure, angesäuert und mit einem mit Wasser praktisch nicht mischbaren organischen Lösungsmittel, wie z. B. Methylenchlorid, extrahiert. Die organische Phase wird eingeengt, das im Rückstand verbleibende Produkt der Formel (I), beispielsweise durch Verreiben mit Alkohol, zur Kristallisation gebracht und durch Absaugen isoliert.

Die nach dem erfindungsgemäßen Verfahren herzustellenden Sulfonylisothioharnstoffe der Formel (I) können als Herbizide eingesetzt werden (vgl. EP-A 5 986).

## Herstellungsbeispiele

### Beispiel 1

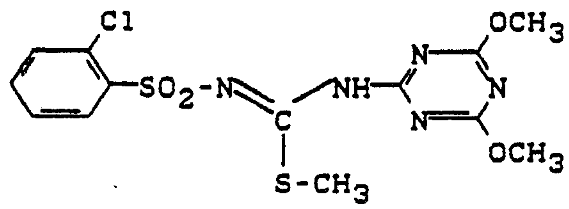

13,0 g (0,05 Mol) N-(4,6-Dimethoxy-s-triazin-2-yl)-iminodithiokohlensäure-S,S-dimethylester werden bei 20 °C zu einer Suspension des Natriumsalzes von 2-Chlor-benzolsulfonsäureamid (0,05 Mol) in 80 ml Dioxan gegeben und das Reaktionsgemisch wird 15 Stunden bei 90 °C bis 100 °C gerührt. Nach Abkühlen auf 20 °C wird mit 2N-Salzsäure angesäuert und mit Methylenchlorid extrahiert. Die Methylenchlorid-Lösung wird eingeengt, der Rückstand mit Ethanol verrieben und das hierbei kristallin angefallene Produkt durch Absaugen isoliert.

Man erhält 13,6 g (68 % der Theorie) N′-(4,6-Dimethoxy-s-triazin-2-yl)-N″-(2-chlor-phenylsulfonyl)-S-methyl-isothioharnstoff vom Schmelzpunkt 159 °C.

Ausgangsstoffe der Formel (III)

Beispiel (III-1)

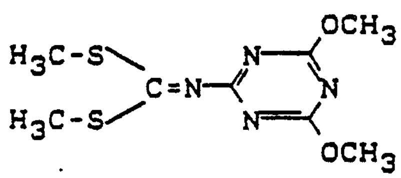

12,5 g (0,22 Mol) Kaliumhydroxidpulver werden portionsweise zu einer Mischung aus 15,4 g (0,10 Mol) 2-Amino-4,6-dimethoxy-s-triazin, 8,5 g (0,11 Mol) Schwefelkohlenstoff und 80 ml Dimethylformamid gegeben, wobei die Innentemperatur unter 35 °C gehalten wird. Das Reaktionsgemisch wird dann 30 Minuten bei 45 °C gerührt. Anschließend werden 31 g (0,22 Mol) Methyliodid unter weiterem Rühren tropfenweise dazu gegeben und das Rühren wird noch 60 Minuten bei 40 °C fortgesetzt. Nach Abkühlen wird mit 250 ml Wasser verdünnt und das hierbei kristallin angefallene Produkt durch Absaugen isoliert.

Man erhält 23,6 g (90 % der Theorie) N-(4,6-Dimethoxy-s-triazin-2-yl)-iminodithiokohlensäure-S,S-dimethylester vom Schmelzpunkt 123 °C.

Beispiel (III-2)

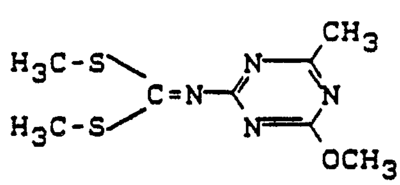

N-(4-Methoxy-6-methyl-s-triazin-2-yl)-iminodithiokohlensäure-S,S-dimethylester der obigen Formel wird analog Beispiel (III-1) in ebenso guter Ausbeute erhalten.
Schmelzpunkt: 78 °C.

**Ansprüche**

1. Verfahren zur Herstellung von Sulfonylisothioharnstoffen der allgemeinen Formel (I)

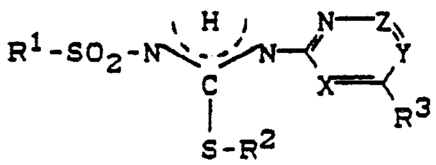

(I)

in welcher
$R^1$ für einen gegebenenfalls substituierten Rest aus der Reihe Alkyl, Aralkyl, Aryl und Heteroaryl steht,
$R^2$ für jeweils gegebenenfalls substituiertes Alkyl oder Aralkyl steht,
$R^3$ für Wasserstoff, Halogen, Hydroxy, Amino oder einen gegebenenfalls substituierten Rest aus der Reihe Alkyl, Alkoxy, Alkylthio, Alkylamino und Dialkylamino steht,
X für Stickstoff oder eine CH-Gruppierung steht,
Y für Stickstoff oder eine $CR^4$-Gruppierung steht, worin
$R^4$ für Wasserstoff, Halogen, Cyano, Alkyl, Formyl, Alkylcarbonyl oder Alkoxycarbonyl steht, und
Z für Stickstoff oder eine $CR^5$-Gruppierung steht, worin
$R^5$ für Wasserstoff, Halogen, Hydroxy, Amino oder einen gegebenenfalls substituierten Rest aus der Reihe Alkyl, Alkoxy, Alkylthio, Alkylamino und Dialkylamino steht,
dadurch gekennzeichnet, daß man Sulfonsäureamide der allgemeinen Formel (II)
$R^1 - SO_2 - NH_2$ (II)
in welcher
$R^1$ die oben angegebene Bedeutung hat,
- oder Metallsalze von Verbindungen der Formel (II)
mit N-Heteroaryl-iminodithiokohlensäure-S,S-diestern der allgemeinen Formel (III)

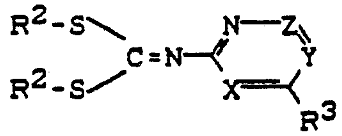

(III)

in welcher
$R^2$, $R^3$, X, Y und Z die oben angegebenen Bedeutungen haben,
gegebenenfalls in Gegenwart eines Säureakzeptors und in Gegenwart eines Verdünnungsmittels bei Temperaturen zwischen 20 °C und 200 °C umsetzt.

2. Verfahren nach Anspruch 1, dadurch gekennzeichnet, daß man bei Temperaturen zwischen 60 °C und 140 °C arbeitet.

3. N-Heteroaryl-iminodithiokohlensäure-S,S-diester der allgemeinen Formel (III)

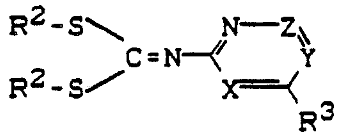

(III)

in welcher
$R^2$ für jeweils gegebenenfalls substituiertes Alkyl oder Aralkyl steht,
$R^3$ für Wasserstoff, Halogen, Hydroxy, Amino oder einen gegebenenfalls substituierten Rest aus der Reihe Alkyl, Alkoxy, Alkylthio, Alkylamino und Dialkylamino steht,
X für Stickstoff oder eine CH-Gruppierung steht,
Y für Stickstoff oder eine $CR^4$-Gruppierung steht, worin
$R^4$ für Wasserstoff, Halogen, Cyano, Alkyl, Formyl, Alkylcarbonyl oder Alkoxycarbonyl steht, und
Z für Stickstoff oder eine $CR^5$-Gruppierung steht, worin
$R^5$ für Wasserstoff, Halogen, Hydroxy, Amino oder einen gegebenenfalls substituierten Rest aus der Reihe Alkyl, Alkoxy, Alkylthio, Alkylamino und Dialkylamino steht.

4. Verfahren zur Herstellung von N-Heteroaryliminodithiokohlensäure-S,S-diestern der allgemeinen Formel (III) gemäß Anspruch 3, dadurch gekennzeicnnet, daß man Aminohetarene der allgemeinen Formel

(IV)

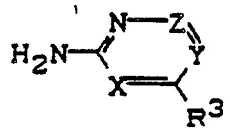

(IV)

in welcher
$R^3$, X, Y und Z die in Anspruch 3 angegebenen Bedeutungen haben,
- oder Metallsalze von Verbindungen der Formel (IV) -
mit Schwefelkohlenstoff (Carbondisulfid) in Gegenwart einer starken Base und in Gegenwart eines Verdünnungsmittels bei Temperaturen zwischen -20 °C und +50 °C umsetzt und in situ weiter mit Alkylierungsmitteln der allgemeinen Formel (V)
$R^2 - X^1$ (V)
in welcher
$R^2$ die in Anspruch 3 angegebene Bedeutung hat und
$X^1$ für Chlor, Brom oder Iod steht,
bei Temperaturen zwischen 0 °C und 100 °C umsetzt.